Europäisches Patentamt

European Patent Office

Office européen des brevets

(11) Veröffentlichungsnummer: **0 223 003**
**A2**

## (12) EUROPÄISCHE PATENTANMELDUNG

(21) Anmeldenummer: 86112610.0

(51) Int. Cl.⁴: **A61M 25/00**

(22) Anmeldetag: 11.09.86

(30) Priorität: 19.11.85 DE 3540949

(43) Veröffentlichungstag der Anmeldung:
27.05.87 Patentblatt 87/22

(84) Benannte Vertragsstaaten:
AT BE CH DE FR GB IT LI LU NL SE

(71) Anmelder: **INTERMEDICAT GMBH**
**Gerliswilstrasse 74**
**CH-6020 Emmenbrücke(CH)**

(72) Erfinder: **Stöber, Herbert**
**Über dem Rötter 1**
**D-3513 Staufenberg 6(DE)**

(74) Vertreter: **von Kreisler, Alek, Dipl.-Chem. et al**
**Deichmannhaus am Hauptbahnhof**
**D-5000 Köln 1(DE)**

(54) Katheter.

(57) Am patientenseitigen Katheterende ist ein Rückschlagventil vorgesehen, das aus dem Ende des
Katheterschlauchs und einem starren Einsatzkörper -
(11) besteht. Der Einsatzkörper (11) ist mit seinem
rückwärtigen Ende (12) im Katheterschlauch (10)
fixiert. An dem Ende (12) führen Umgehungskanäle
vorbei. Das vordere Ende des Einsatzkörpers (11) ist
als zylindrischer Stopfen (13) ausgebildet, an dessen
Umfangsfläche sich das als Ventilschlauch wirkende
Katheterende anlegt. Bei einem Überdruck im
Katheterschlauch (10) hebt das Schlauchende von
dem Stopfen (13) ab und das Druckmedium kann
entweichen.

FIG.1

## Katheter

Die Erfindung betrifft einen Katheter mit einem Katheterschlauch mit an der Spitze vorgesehem Rückschlagventil, welches das Eindringen von Flüssigkeit verhindert, bei innerem Überdruck jedoch öffnet.

Ein bekannter Katheter mit Rückschlagventil dieser Art (EP-PS 0 018 179) weist eine elastische rohrförmige Hülse auf, die die Katheterspitze seitlich umgibt. Das Ende des Katheterlumens ist mit einem Stopfen fest verschlossen, der durch einen seitlichen Ansatz in einem Loch des Katheterschlauchs verankert ist. Die elastische Hülse überdeckt seitliche Löcher des Katheterschlauchs. Bei einem Überdruck im Katheterlumen hebt die elastische Hülse stellenweise von dem Katheterschlauch ab, so daß Flüssigkeit vom Inneren des Katheterschlauchs durch die aufgeweitete elastische Hülse nach außen fließen kann. Das Eindringen von Flüssigkeit in die Katheterspitze wird durch die abdichtende Wirkung der elastisch an dem Katheterschlauch anliegenden Hülse verhindert. Ein derartiges Rückschlagventil ist aufwendig in der Herstellung, weil zum Verschließen des Katheterendes ein separates Verschlußstück erforderlich ist, das in bezug auf den Katheterschlauch abdichten muß, und weil an dem Katheterschlauch radiale Öffnungen vorgesehen werden müssen. Durch das Zusammenwirken zweier elastischer Teile, nämlich des Katheterschlauchs und der Hülse, sind ein definiertes Dichtungsverhalten und Öffnungsverhalten des Ventils nur schwer zu erreichen. Außerdem ist es schwierig, bei der Montage des Ventils die elastische Hülse über den ebenfalls elastischen Katheterschlauch zu ziehen.

Der Erfindung liegt die Aufgabe zugrunde, einen Katheter der eingangs genannten Art zu -schaffen, der auf einfache Weise herstellbar ist und bei dem das Rückschlagventil ein definiertes Betriebsverhalten hat.

Die Lösung dieser Aufgabe besteht erfindungsgemäß darin, daß das Rückschlagventil einen langgestreckten starren Einsatzkörper aufweist, der an seinem rückwärtigen Ende am Katheterschlauch fixiert ist, daß mindestens ein an dem rückwärtigen Ende des Einsatzkörpers vorbeiführender Umgehungskanal vorgesehen ist und daß das vordere Ende des Einsatzkörpers als Stopfen ausgebildet ist, der von dem Katheterschlauch zur Bildung des Ventilauslasses abdichtend elastisch umspannt ist.

Bei dem erfindungsgemäßen Katheter befindet sich das gesamte Rückschlagventil innerhalb der Außenkontur des Katheterschlauchs und es sind im Bereich des Rückschlagventils keine radialen Löcher im Katheterschlauch vorhanden. Die elastischen Eigenschaften des Katheter schlauchs werden zur Bildung der Ventilhülse ausgenutzt, die sich bei einem Innendruck aufweitet. Die Elastizität des Katheterschlauchs ist mit dem Übermaß, das der Einsatzkörper in bezug auf den Innendurchmesser des Katheterschlauchs hat, so abgestimmt, daß bei einem relativ geringem Innendruck im Katheterschlauch das Ventil öffnet, d.h. daß der Katheterschlauch mindestens abschnittsweise von dem Stopfen abhebt. Die Katheterspitze selbst bildet somit den äußeren Ventilschlauch des Rückschlagventils. Die Ventilöffnung befindet sich am vorderen Ende des Katheterschlauchs.

Ein besonderer Vorteil besteht darin, daß ein starrer Einsatzkörper verwendet wird, so daß der elastische Katheterschlauch mit einem starren Teil zusammenwirkt und dieses starre Teil fest umschließt. Dadurch wird ein reproduzierbares Ventilverhalten erzielt. Zur Bildung des Rückschlagventils wird nur ein einziges Zusatzteil, nämlich der Einsatzkörper, benötigt. Der starre Einsatzkörper kann relativ leicht in den Katheterschlauch eingeschoben werden; es ist nicht erforderlich, zwei flexible Schläuche übereinanderzuziehen. Vorteilhaft ist ferner, daß der Außendurchmesser des Katheters im Bereich des Rückschlagventils nicht oder nur in ganz geringem Maße vergrößert wird.

Bei einer bevorzugten Ausführungsform der Erfindung ist vorgesehen, daß ein Mittelteil zwischen dem rückwärtigen und dem vorderen Ende des Einsatzkörpers radiale Vorsprünge zum Abstützen des Katheterschlauchs unter Freihaltung von Längskanälen aufweist. Hierbei dient der rückwärtige Endbereich des Einsatzkörpers als Befestigungsteil, das jedoch das Katheterlumen nicht vollständig blockiert. Der Durchmesser des Mittelteils des Einsatzkörpers ist geringer als derjenige des Katheters, jedoch können an dem Mittelteil radial abstehende Rippen zum Abstützen des Katheters vorgesehen sein.

Zweckmäßigerweise ist das vordere Ende des Einsatzkörpers als eine das Ende des Katheterschlauchs überragende abgerundete Kuppe ausgebildet. Die Infusionsflüssigkeit tritt um die Kuppe herum aus dem Ende des Katheterschlauchs aus. Andererseits bildet die Kuppe des Einsatzkörpers das vordere Ende des gesamten Katheters. Durch die abgerundete Kuppenform werden Verletzungen beim Einführen des Katheters in den Körper des Patienten vermieden.

Im folgenden wird unter Bezugnahme auf die Zeichnungen ein Ausführungsbeispiel der Erfindung näher erläutert.

Es zeigen:

Fig. 1 einen Längsschnitt durch das an der Katheterspitze befindliche Rückschlagventil,

Fig. 2 einen Schnitt entlang der Linie II-II von Fig. 1,

Fig. 3 einen Schnitt entlang der Linie III-III von Fig. 1 und

Fig. 4 einen Schnitt entlang der Linie IV-IV von Fig. 1.

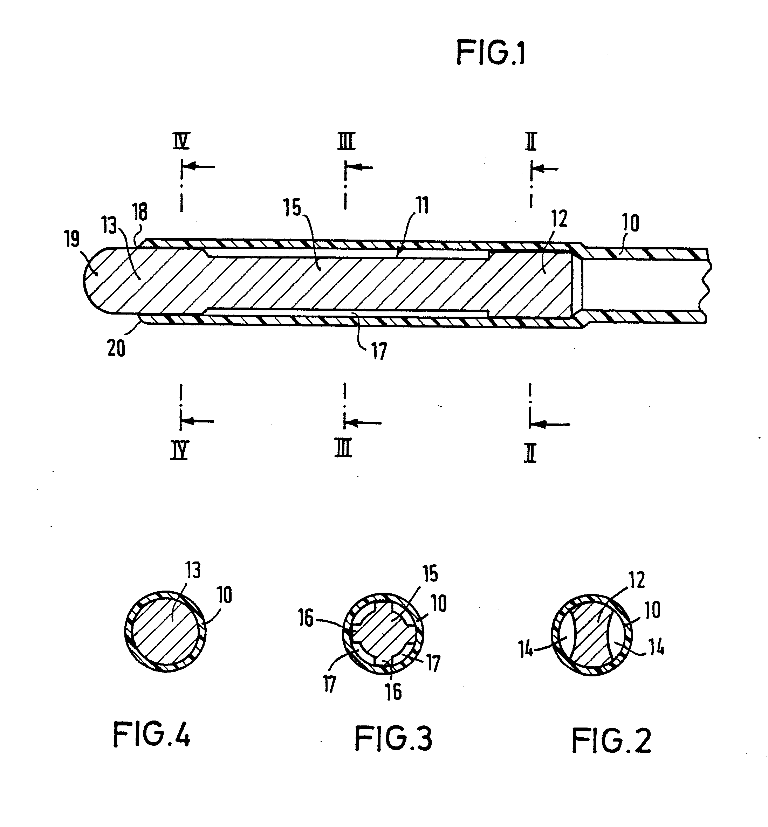

Von dem Katheter ist in der Zeichnung nur das patientenseitige (vordere) Katheterende dargestellt. Der Katheterschlauch 10 besteht aus einem gewebeverträglichen elastischen Material. In das vordere Ende des Katheterschlauchs 10 ist der Einsatzkörper 11 eingeschoben. Der längliche Einsatzkörper 11 weist an seinem rückwärtigen Ende einen Befestigungsabschnitt 12 auf und ist an seinem vorderen Ende als Stopfen 13 ausgebildet. Das rückwärtige Ende 12 hat den in Fig. 2 dargestellten Querschnitt. Das Ende 12 ist im wesentlichen zylindrisch, wobei jedoch an entgegengesetzten Seiten Abflachungen zur Bildung von Längskanälen 14 zwischen dem Einsatzkörper und der Wand des Katheterschlauchs 10 vorgesehen sind. Die mit dem Katheterschlauch 10 in Berührung stehenden Umfangsbereiche des Endes 12 weiten den Katheterschlauch 10 geringfügig auf, so daß durch Reibung eine Lagesicherung des Einsatzkörpers 11 im Inneren des Katheterschlauchs 10 erfolgt. Erforderlichenfalls kann die Fixierung des Einsatzkörpers 11 im Katheterschlauch auch durch Kleben oder eine andere Verbindungstechnik erfolgen.

Zwischen dem Ende 12 und dem vorderen Stopfen 13 erstreckt sich der Mittelteil 15 des Einsatzkörpers 11. Im Mittelteil 15 hat der Einsatzkörper 11 gemäß Fig. 3 einen Querschnitt, der kleiner ist als der Querschnitt des Katheterlumens. Von einem zylindrischen Kern stehen radiale Stege 16, die umfangsmäßig verteilt angeordnet sind, radial ab. Die Stege 16 dienen zur seitlichen Abstützung der Wand des Katheterschlauchs 10. Zwischen den Stegen 16 befinden sich Längskanäle 17, durch die Infusionsflüssigkeit fließen kann.

Der Stopfen 13, der das vordere Ende des Einsatzkörpers 11 bildet, ist zylindrisch und an seiner Außenfläche liegt das vordere Ende des Katheterschlauchs 10 unter radialer Spannung an. Der Katheterschlauch 10 ist an dem Stopfen 13 nicht oder nur stellenweise fixiert, so daß er mindestens auf einem Teilbereich des Umfangs des Stopfens 13 von diesem abheben kann, wenn im Inneren des Katheterschlauchs ein Überdruck auftritt. Das Druckmedium kann dann durch das vordere Ende 18 hindurch um den Stopfen 13 herum entweichen.

Der Stopfen 13 überragt das vordere Ende 18 des Katheterschlauchs und er ist an seinem Ende als abgerundete Kuppe 19 ausgebildet.

Der Einsatzkörper 11 ist einstückig, z.B. aus Kunststoff, gefertigt. Er besteht aus Vollmaterial. Der größte Außendurchmesser des rückwärtigen Bereichs 12 und des mittleren Bereichs 15 ist im wesentlichen gleich dem Außendurchmesser des Stopfens 13. Dieser Außendurchmesser ist geringfügig größer als der Innendurchmesser des Katheterschlauchs 10, so daß der Katheterschlauch durch den Einsatzkörpers 11 geringfügig aufgeweitet wird.

Die vordere Kante 20 des Katheterschlauchs 10 ist nach außen abgeschrägt, so daß sie eine sich an den Stopfen 13 anschmiegende Lippe bildet. Dadurch wird die Dichtwirkung gegen äußeren Druck erhöht.

Eine Infusionslösung, die dem Patienten zugeführt werden soll, wird in den Katheter eingeführt und strömt durch die Längskanäle 14 an dem rückwärtigen Ende 12 des Einsatzkörpers vorbei in die Längskanäle 17 des Mittelteils 15 hinein. Der im Katheter herrschende Druck hebt das vordere Ende des Katheterschlauchs von dem Stopfen 13 ab, so daß die Infusionslösung endseitig am Katheterschlauch austreten kann. Andererseits kann Körperflüssigkeit wegen der Dichtwirkung des den Stopfen 13 umschließenden vorderen Katheterendes nicht in den Katheterschlauch eindringen.

## Ansprüche

1. Katheter mit einem Katheterschlauch (10) mit an der Katheterspitze vorgesehenem Rückschlagventil, welches das Eindringen von Flüssigkeit verhindert, bei innerem Überdruck jedoch öffnet,

**dadurch gekennzeichnet,**

daß das Rückschlagventil einen langgestreckten starren Einsatzkörper (15) aufweist, der an seinem rückwärtigen Ende (12) am Katheterschlauch (10) fixiert ist, daß mindestens ein an dem rückwärtigen Ende (12) des Einsatzkörpers (11) vorbeiführender Umgehungskanal (14) vorgesehen ist und daß das vordere Ende des Einsatzkörpers (11) als Stopfen (13) ausgebildet ist, der von dem Katheterschlauch zur Bildung des Ventilauslasses abdichtend umspannt ist.

2. Katheter nach Anspruch 1, dadurch gekennzeichnet, daß ein Mittelteil (15) zwischen dem rückwärtigen und dem vorderen Ende des Einsatzkörpers (11) radiale Stege (16) zum Abstützen des Katheterschlauchs (11) unter Freihaltung von Längskanälen (17) aufweist.

3. Katheter nach Anspruch 1 oder 2, dadurch gekennzeichnet, daß das vordere Ende des Einsatzkörpers (11) als eine das Ende des Katheterschlauchs (10) überragende abgerundete Kuppe (19) ausgebildet ist.

4. Katheter nach einem der Ansprüche 1 bis 3, dadurch gekennzeichnet, daß das rückwärtige Ende (12) des Einsatzkörpers (11) etwa den gleichen maximalen Durchmesser hat wie der Stopfen (13) und seitliche Abflachungen oder Nuten aufweist.

5. Katheter nach einem der Ansprüche 1 bis 4, dadurch gekennzeichnet, daß die vordere Kante (20) des Katheterschlauchs (10) nach außen abgeschrägt ist.

## FIG.1

## FIG.4

## FIG.3

## FIG.2